# EUROPEAN PATENT APPLICATION

(11) **EP 1 067 194 A1**
(43) Date of publication of application: **10.01.2001**
(21) Application number: 99107611.8
(22) Date of filing: 16.04.1999
(51) Int. Cl.: C12N 15/86, A61K 48/00, C07K 14/705

(54) **Vectors containing a gene coding for CD40 and/or CD40L under the control of a cytokine-inducible promoter which is a human acute phase amyloid A gene promoter. Methods for their production and uses thereof**

(71) Applicant: F. HOFFMANN-LA ROCHE AG, 4070 Basel (CH)
(72) Inventor: The designation of the inventor has not yet been filed
(74) Representative: Schreiner, Siegfried, Dr.

(57) **Abstract**

A vector for the expression of a nucleic acid encoding at least one polypeptide with CD40 and/or CD154 activity, wherein the expression of said nucleic acid is under the control of a cytokine-inducible promotor, is preferably administered locally to solid tumors for tumor therapy, wherein the promotor is a human acute phase amyloid A gene promotor.

## Description

The invention relates to expression vectors for CD40 and/or CD40L (CD154), wherein the expression is under the control of a cytokine-inducible promoter, to methods for their production, pharmaceutical compositions containing said vectors, and to uses thereof.

Common gene therapy vectors use exogenous viral promoters, such as Simian virus 40, Rous sarcoma virus and cytomegalovirus, for expression of therapeutically useful proteins. Such promoters are constitutive promoters and they would require specific inducing signals. From Varley, A.W., Proc. Natl. Acad. Sci. USA 92 (1995) 5346-5356, U.S. Patent No. 5,744,304 and U.S. Patent No. 5,851,822 there is known an inflammation-induced recombinant protein expression in vivo using promoters from acute phase protein genes which are inducible by cytokines. The authors suggest that gene delivery vectors that utilize such promoters for the control of the production of antiinflammatory proteins, such as the IL-1 receptor antagonist, TNF receptor, IL-4, IL-10, adrenocorticotropic hormone or prostaglandin synthase, could prove useful for modulating the intensity and duration of the inflammatory response in a manner that is regulated by natural homeostatic mechanisms. It is further known from WO 98/40506 that gene therapy vectors containing genes coding for cytokines such as IL-2 which are under the control of the cytokine-inducible promoter acute phase serum amyloid A gene promoter are useful for tumor therapy.

One of the primary changes that occurs during the acute phase response to inflammation is the alteration of the synthetic profile of the liver which produces increased amounts of acute phase reactants (APRs) (Fey, G.H., and Gauldie, J., In: H. Popper and F. Schaffner (Eds.), Progress in Liver Diseases. Vol.9. (1989) WB Saunders, Philadelphia, p.89). This is triggered by pro-inflammatory molecules (e.g. IL-1 and TNF) which act via receptor-mediated signal transduction events to activate transcription factors (e.g. NF-kB). These in turn bind to specific regions in the promoters of APR genes and induce transcription. Acute phase serum amyloid A (A-SAA) is a major APR whose concentration increases by up to 1000 fold during the acute phase response (Hoffman, J.S., and Benditt, E.P., J. Biol. Chem. 257 (1982) 10510; Marhaug, G., Scand. J. Immunol. 18 (1983) 329).

Human A-SAA is the product of two genes, SAA1 and SAA2, which are almost identical with respect to their organization, sequence and mode of expression (Sipe, J.D., et al., Biochemistry 24 (1985) 2931; Woo, P., et al., J. Biol. Chem. 262 (1987) 15790-15795; Dwulet, F.E., et al., Biochemistry 27 (1988) 1677; Beach, C.M., et al., Biochem. J. 282 (1992) 615). A-SAA levels increase by as much as 1000-fold during the acute phase response (Hoffman, J.S., and Benditt, E.P., J. Biol. Chem. 257 (1982) 10510; Marhaug, G., Scand. J. Immunol. 18 (1983) 329). Although this dramatic induction by inflammatory stimuli indicates an important protective role for A-SAA in the short term, the long term synthesis of high levels of A-SAA in chronic inflammation has negative clinical consequences. A-SAA is the serum precursor of amyloid A which is the principal constituent of the insoluble fibrils deposited in organs and tissues in the progressive, fatal condition secondary or reactive systemic amyloidosis (reviewed by Steel, D.M., and Whitehead, A.S., Immunol. Today 15 (1994) 81).

The promoters of a subset of these genes, those encoding the major APRs (in humans the acute phase serum amyloid As [A-SAAs] and C-reactive protein [CRP]), are extremely responsive to such signals causing them to be massively induced during the acute phase response (Kushner, I., Ann. NY Acad. Sci. USA 389 (1982) 39; Kushner, I., and Mackiewicz, A., Disease Markers 5 (1987) 1; Fey, G.H., and Gauldie, J., In: H. Popper and F. Schaffner (Eds.), Progress in Liver Diseases. Vol.9. (1989) WB Saunders, Philadelphia, p.89). Consequently major APR promoters have the potential to be used as indicators of the ability of naturally occurring and synthetic molecules to act as pro- and antiinflammatory reagents. They also could be adapted to drive the synthesis of products of heterologous genes, both in vivo for gene therapy and in vitro for the large scale synthesis of recombinant proteins.

The human acute-phase serum amyloid A gene together with its corresponding promoter (SAA2-promoter) have been cloned by Uhlar et al., J. Immunol. Methods 203 (1997) 123-130. The SAA2-promoter is described to be active in inflammed tissue and can be highly activated (by factor 70) in vitro by monocyte conditioned medium as well as IL-1β + IL-6 and TNF-α + IL-6. IL-1β mediated stimulation can be blocked by IL-1 receptor antagonist (Uhlar et al., J. Immunol. Methods 203 (1997) 123-130 and Steel et al., Biochem. Journal 291 (1993) 701-707). However it is inactive in non-inflammed, normal tissues as well as in cell lines without the addition of the pro-inflammatory cytokines.

CD40, a cell surface receptor from the family of TNF receptors, was first identified and functionally characterized on B lymphocytes, where it is involved in the regulation of growth and differentiation. It may also be found, however, on other cell types, for example on T cells (activation → proliferation stimulation, cytokine secretion), dendritic cells, and monocytes (activation → expression of costimulatory molecules, secretion of inflammatory cytokines, e.g., TNF-α, IL-8, IL-12) as well as on carcinomas (activation leads to inhibition of growth) (van Kooten et al., Int. Arch. Allergy Immunol. 113 (1997) 393-399). It is a member of the group of type I membrane proteins, which implies an extracellular N terminus, a transmembrane region, and an intracellular C terminus (Laman et al., Critical Reviews in Immunology 16 (1996) 59-108).

CD154 (= CD40L or gp39) is the ligand of CD40 (WO 93/08207). It tends to be expressed on activated CD4+ T cells, but is also found on activated B cells and dendritic cells. Interaction between CD40L and CD40 plays a central role in the development of both humoral and cellular immunity. Ligation of CD40L to CD40 initiates both a CD40-mediated signal (see above, APC (Antigen Presenting Cell) activation) and a direct T-cell-stimulating signal via CD40L itself (T cell activation). CD40L belongs to the group of type II membrane proteins, which implies an intracellular N terminus, a transmembrane region and an extracellular C terminus (Laman et al., Critical Reviews in Immunology 16 (1996) 59-108). There have also been described soluble forms of CD40L, which means that they only consist of the C terminal extracellular domain and are capable of activating CD40. Consequently, the extracellular domain is sufficient for the formation of trimers, which, in turn, bind to the extracellular domain of the receptor CD40 and thereby trimerize and activate this receptor. Yet, qualitative differences have been discussed in the literature with respect to the mediation of signals by soluble CD40L and by membrane-bound CD40L (Laman et al., Critical Reviews in Immunology 16 (1996) 59-108).

It is the object of the invention to provide new gene therapy vectors which are particularly useful for local treatment of solid tumors.

### Summary of the Invention

According to the invention it is possible to induce the presentation of tumor-specific antigens on immunocompetent cells and to induce differentiation and proliferation of such immunocompetent cells like T cells, macrophages and dendritic cells.

The invention comprises a vector for the expression of a nucleic acid encoding at least one polypeptide with CD40 and/or CD154 activity, wherein the expression of said nucleic acid is under the control of a cytokine-inducible (inflammatory regulated) promoter, preferably under the control of an acute phase protein gene promoter.

The invention further comprises, in a preferred embodiment, a vector or a combination of vectors according to the invention which expresses, in addition, one or more genes selected from the group consisting of the genes encoding IL-2, IL-12 and Interferon-alpha.

If two or more genes are used, then said genes can be under the control of the same promoter on the same vector, under the control of two identical or different promoters on the same vector, or on different expression vectors, whereby at least one gene encoding CD40 or CD40L is under the control of a cytokine-inducible promoter. Such a co-expression of CD40/CD40L ensures a constitutive activation of the CD40 receptor which avoids and eliminates anergism against tumor cells.

The invention preferably comprises a vector for the expression of genes coding for polypeptides with CD40 and/or CD40L (CD154) activity, wherein the expression of said genes is under the control of the human acute phase serum amyloid A gene promoter (SAA1or SAA2, hereafter referred to as "SAA promoter").

The invention further comprises pharmaceutical compositions containing at least one expression vector according to the invention as an essential component.

In a preferred embodiment of the invention, the pharmaceutical composition contains at least two genes on one or more vectors. Preferred examples of genes encoded by such vectors are listed below in Tables 1 and 2.

**Table 1**

| **Vector 1** | **Vector 2** |
|---|---|
| CD40 | CD154 |
| CD40 | IL-12, IL-2 and/or interferon-α |
| CD154 | IL-12, IL-2 and/or interferon-α |
| CD40/CD154 | IL-12, IL-2 and/or interferon-α |

Genes from Vector 1 and Vector 2 can also be co-expressed on one vector.

The following combinations are also preferred:

**Table 2**

| **Vector 1** | **Vector 2** | **Vector 3** |
|---|---|---|
| CD40 | CD154 | IL-12 and/or IL-2 |
| CD40 | IL-2 | IL-12 |
| CD154 | IL-2 | IL-12 |
| CD154 | IL-2 and IL-12 | - |
| CD40/CD154 | IL-2 and IL-12 | - |
| CD40/CD154 | IL-2 | IL-12 |

Genes fromVectors 1 to 3 can also be combined on one or two vectors.

Particularly preferred is the combination of CD154 with IL-12 (IL-12 as gene or as protein).

The invention further comprises methods for the production of such expression vectors and of pharmaceutical compositions containing such vectors. The pharmaceutical compositions are preferably used for in vivo treatment of tumor cells of a patient (gene therapy treatment). According to the invention it was found that vectors containing the CD40/CD40L gene under the control of the SAA promoter are preferred and improved therapeutic agents for the treatment of tumor diseases.

A preferred construct according to the invention was generated by cloning an SAA promoter upstream of a CD40 and/or a CD40L gene.

One may introduce the disclosed nucleic acid segments into a cell or a mammalian host by any of several means, including vector transfection. Viral vectors may be used to infect human and animal cells with the recombinant DNA; certain adenoviral vectors have proved particularly useful. Adenoviral vectors are preferred. Of course DNA segments need not be introduced into cells by a viral vector: direct transformation may be performed by electroporation, gene gun techniques, or DNA-liposome complexes, for example. DNA/liposome complexes have been used to introduce DNA encoding prostaglandin synthase into rabbits, with subsequent production of prostaglandin E2 and prostacyclin (Conary et al., J. Clin. Invest. 93 (1994) 1834-1840).

An appropriate vector includes the gene encoding the selected protein or proteins such that the gene is, or the genes are, under the transcriptional control of a cytokine-responsive promoter of an acute phase protein gene promoter. The vector is introduced into a host cell by any of a number of procedures known to those skilled in the art, such as direct introduction of DNA by gene gun techniques, liposomal transfection or direct local injection. Direct infusion is preferred in the case of bladder carcinomas and infusion with an endoscopic probe is preferred in the case of colon carcinomas.

The invention is not limited to the use of vectors that respond by producing a single polypeptide. For example, multiple constructs may be administered, each producing different polypeptides selected from the group consisting of CD40, CD154, IL-2, IL-12 and Interferon-alpha; alternatively, multiple constructs may be combined in a single vector to produce several polypeptides or fused proteins that may or may not be engineered with cleavage sites to provide individual polypeptides in vivo.

In a further preferred embodiment of the invention, the expression vectors according to the invention are combined with polypeptides with the activity of Interleukin-2 (IL-2), Interleukin-12 (IL-12) and/or Interferon-alpha and/or 5-fluorouracil for application in vivo.

### Detailed Description of the Invention

Cytokine-inducible promoters according to the invention are promoters which are responsive to an inflammation cascade, such as an acute phase protein gene promoter or one responsive to IL-1, IL-6 or TNF. Preferred acute phase protein gene promoters are sPLA₂-promoter or the serum amyloid A2 promoter. Of course other acute phase protein gene promoters could be used, including promoters of C-reactive protein, fibrinogen, serum amyloid protein, complement factor 3, orosomucoid, alpha.sup.1 -antiprotease (antitrypsin) and other isoforms of SAA.

Promoters useful in the invention are highly sensitive to cytokine induction during local or systemic inflammation due to the action of pro-inflammatory proteins like IL-1β, IL-6 and TNFα. Also due to the fact that expression of these cytokines has been found in several tumors (e.g., squamous cell carcinomas) but not in normal uninflamed tissue, a specific expression of such promoters in these types of tumors can be found. Several tumors have been described to express high amounts of pro-inflammatory cytokines, e.g. Knerer et al., Acta Oto-Laryngologica 116 (1996) 132-136 described high level expression of IL-1 and TNF-α in squamous cell carcinoma of the head and neck. But also other tumors have been described to express pro-inflammatory cytokines, e.g. Levy et al., Neurosurgery 39 (1996) 823-823 found IL-6 and IL-β expression in meningonomas. Cytokine-inducible promoters such as the preferred SAA2-promoter will be activated in tumor tissues such as melanomas, prostate tumors, bladder carcinomas, breast tumors and colon tumors. They are therefore particularly suitable for incorporation into constructs designed to drive the synthesis of desired polypeptides. The invention is especially advantageous for the treatment of squamous cell carcinomas like head and neck cancers.

A promoter or an expression control region according to the invention is understood as a nucleic acid region which causes the expression of DNA and hence transcription into mRNA and which usually has a length of 0.5-5 kb. Such expression control regions usually contain enhancer regions and promoter regions to which transcription factors or repressors can bind. Expression control regions can be regulated via binding of activating or repressing factors. A regulatory region according to the invention is understood as a region which influences expression due to induction by cytokines. Based on this, the expression is stimulated.

In a preferred embodiment of the invention, a minimal promoter combined with enhancing elements is used. Minimal promoters and methods for their construction are described, for example, in Luckow, B., et al., Nucleic Acids Res. 10 (1987) 5490 and Spear, B.T., et al., DNA Cell Biol. 14 (1995) 635-642. A minimal promoter useful in the expression vectors according to the invention contains at least a TATA-box, one ore more cytokine-responsive elements (CRE) and one or more binding sites for transactivators such as NFkB, CEBP/NF-IL6 and also others such as YY1, SAF and AP1. Such binding sites usually consist of 4 to 12 nucleotides in length. CREs are described, for example, in Dendorfer, U., Artif. Organs 20 (1996) 437-444; Birt, D.F., et al., J. Nutr. 129 (1999) 25 Suppl., 5715-5745; Kang, D.C., et al., Int. J. Oncol. 13 (1998) 1117-1126; Weber-Nordt, R.M., et al., Leuk. Lymphoma 28 (1998) 459-467; Sen, C.K., FASEB J. 10 (1996) 709-720.

It is further preferred to use within the vectors according to the invention a translation control element within the SAA2 5'-UTR that plays a crucial role in modulating A-SAA production. This element is a cell- and/or tissue-specific translational enhancer. Its efficiency could be mediated by an intracellular factor that is activated or synthesized de novo after cytokine treatment. The sequence of this enhancer element is shown in SEQ ID NO:4 of WO 98/40506. Preferably, the enhancer is used in conjunction with the A-SAA promotor (i.e., downstream of the promotor and upstream of the gene encoding the product of interest). It is named "SAA enhancer". By "SAA enhancer" according to the invention there is understood an enhancer which has the function of an SAA enhancer and is essentially identical to said SEQ ID NO:4. Also preferred are enhancers with DNA sequences which hybridize with said SEQ ID NO:4 under stringent conditions and have the ability to act as an enhancer element.

The phrase "hybridize under stringent conditions" means that two nucleic acid fragments are capable of hybridization to one another under standard hybridization conditions described in Sambrook et al., "Expression of cloned genes in E.coli" in Molecular cloning: a laboratory manual (1989), Cold Spring Harbor Laboratory Press, New York, USA, 9.47-9.63 and 11.45-11.61. More specifically, "stringent conditions" as used herein refers to hybridization in 6.0 x SSC at about 45°C followed by a wash of 2.0 x SSC at 50°C. For a selection of the stringency the salt concentration in the wash step can be selected, for example, from about 2.0 x SSC at 50°C for low stringency to about 0.2 x SSC at 50°C for high stringency. In addition, the temperature in the wash step can be increased from low stringency conditions at room temperature, about 22°C, to high stringency conditions, about 65°C.

The SAA2 promoter which is preferred according to the invention is a promoter which is very silent in healthy noninflammated tissues but which is specifically active in inflammated but also in tumor tissue. A murine squamous carcinoma cell line like SCC-VII is a model system to show the functionality and tumor specificity of the SAA2 promoter in tumors expression preinflammatory cytokines as it is already described for human squamous carcinomas.

"SAA promoter" according to the invention therefore means a promoter which has the function of an A-SAA promoter and which is therefore inducible by a cytokine in substantially the same manner as the SAA promoter described in WO 98/40506 and which is essentially identical to the sequence of the SAA1 and/or SAA2 promoter. Also preferred are promoters which are coded by DNA sequences which hybridize with SEQ ID NO:1 shown in WO98/40506 under stringent conditions and have the ability to act as an expression control sequence inducible by cytokines. In view of the fact that there is only a 10% difference in sequence between the SAA1 promoter and the SAA2 promoter, it is obvious that such a difference in sequence does not have a substantial influence on the functionality of the SAA promoter, and that such variations therefore are not essential for the function.

The term "under the control" means that the promoter is located in a position relative to that of the DNA encoding the desired polypeptide that allows the promoter to efficiently direct transcription of the structural gene or the genes for the desired polypeptide(s).

According to the invention it is preferred to use an expression system for CD40 and/or CD154 (CD40L). If the tumor cells to be treated are characterized as CD40", it is preferred to use an expression system for CD40. If the tumor cells to be treated are characterized as CD40⁺, it is preferred to use an expression system for CD40L. It was surprisingly found, however, that even for CD40⁺ tumor cells an additional expression of CD40 results in an increase in the level and length of the activity phase of tumor-specific cytolytic T cells.

CD40 according to the invention is understood as a polypeptide with the activity and biochemical characteristics of CD40. Such polypeptides are, for example, human or murine CD40 or modifications thereof such as mutations, deletions or substitutions. Preferred modified CD40 polypeptides are at least 80% homologous to human CD40 preferably within the extracellular domain. Homology according to the invention can be determined with the aid of the computer programs Gap or BestFit (University of Wisconsin; Needleman and Wunsch, J. Mol. Biol. 48 (1970) 443-453; Smith and Waterman, Adv. Appl. Math. 2 (1981) 482-489).

The CD40 molecule is 277 amino acids in length (MW = 30,619 Da) and starts with a hydrophobic signal sequence of a length of 19 amino acids (Pos. 1-19), followed by an extracellular domain of a length of 174 amino acids (Pos. 20-193), a hydrophobic transmembrane domain of a length of 22 amino acids (Pos. 194-215), and a cytoplasmic domain of a length of 62 amino acids (Pos. 216-277) (Swiss-Prot.: P25942 and Stamenkovic et al., The EMBO J. 8 (1989) 1403-1410). The extracellular domain is responsible for the ligand binding and for the thereby mediated trimerization of the active complex. The cytoplasmic domain mediates the signal transduction of the complex that is activated by trimerization. Signal transduction is mediated by the socalled TRAF proteins (TNF-R Associated Factor), whose binding sites are located in the cytoplasmic domain of CD40, and ultimately leads to the activation of various transcription factors, NF-κB being the most prominent of these transcription factors. The analogous murine CD40 molecule is 289 amino acids in length (MW = 21,111 Da) (Grimaldi et al., The J. of Immunol. 149 (1992) 3921-3926 as well as Swiss-Prot.: P27512) and has a signal sequence of a length of 19 amino acids (Pos. 1-19), followed by an extracellular domain of a length of 174 amino acids (Pos. 20-193), a hydrophobic transmembrane domain of a length of 22 amino acids (Pos. 194-215), and a cytoplasmic domain of a length of 74 amino acids (Pos. 216-289).

CD154 according to the invention is understood as a polypeptide with the activity and biochemical characteristics of CD154. Such polypeptides are, for example, human or murine CD154 or modifications thereof such as mutations, deletions or substitutions. Preferred modified CD154 polypeptides are at least 80% homologous to human CD154 preferably within the extracellular domain.

The human CD154 protein is 261 amino acids in length (Swiss-Prot.: P29965, MW = 29,273 Da) and starts with a cytoplasmic region of a length of 22 amino acids (Pos. 1-22), followed by a signal anchor of a length of 24 amino acids (type II membrane protein; one signal peptide is missing!) (Pos. 23-46), and an extracellular, signal-mediating domain of a length of 215 amino acids (Pos. 47-261). The mature glycoprotein has a molecular weight of 35 kDa. The murine CD40L (Swiss-Prot.: P27548) is 260 amino acids in length (MW = 29,396 Da) and starts with a cytoplasmic region of a length of 22 amino acids (Pos. 1-22), followed by a signal anchor of a length of 24 amino acids (Pos. 23-46) and an extracellular, signal-mediating domain of a length of 214 amino acids (Pos. 47-260). The mature glycoprotein has a molecular weight of 33 kDa.

The three-dimensional structure of CD40L has been elucidated: The structure of the CD40 / CD40L complex has been derived from the known homologous structure of TNF-R / TNF-β. This has led to the model according to which CD40L binds as a homotrimer to a homotrimer of CD40. Human CD40 and murine CD40 exhibit 76% homology. Human CD40L and murine CD40L exhibit 86% homology.

In a preferred embodiment, the genes for the human or murine (or chimeric from both species) CD40 and CD40L are being co-expressed on the same or on different vectors. This can be effected by means of cotransfection or cotransduction of two viral or plasmid vectors which carry both genes or of a single vector on which both genes are present in coded form. In the latter embodiment, both genes can be expressed by separate promoters (the promoters may be identical or different from one another), they may be present coupled via an IRES sequence (internal ribosomal entry site) in the expression, or coupled via a splice-donor sequence before the first gene and a splice-acceptor sequence after the first gene, that is, before the second gene. In the latter embodiment, in the case of unprocessed mRNA, the first gene would be read, and in the case of processed mRNA, the first gene would be spliced out and the second gene would be read. Such a co-expression of CD40 and CD40L ensures a constitutive activation of CD40L also in CD40L⁻ tumor cells.

Genes encoding CD40 and CD40L (= CD154) or combinations or chimerics of both represent ,,immunological master genes", that is to say, after ligand binding through CD40L, CD40 induces a great number of co-stimulatory factors, inflammation-inducing or maintaining factors, and thus, a strong immune reaction. It is further preferred to use, in addition, genes which code for IL-12, IL-2 and other immunostimulatory factors, or combinations thereof, or the related polypeptides. CD40 also induces the NFkB transduction pathway, at the end of which there are a great number of inflammation mediators such as, for instance, SAA2. The preferred embodiment of the invention therefore consists of a CD40 or CD40L gene or combinations or chimerics of both under the expression control of the cytokine-inducible promoter. It is an advantage of this embodiment that, after being inserted into a tumor, which even expresses pro-inflammatory cytokines only to a little extent, it induces an autocrine activation cascade, i.e., the inflammatory cytokines will induce the promoter on the inserted vector, which will express the CD40 or CD40L gene or combinations or chimerics of both, and CD40, in turn, will activate the NFkB transduction pathway, at the end of which there is the activation of the promoter. This effect can be further enhanced by inserting into the cytokine-inducible promoter the binding sites for pro-inflammatory transactivators, such as NFkB or NF-IL6 (e.g., the region from -190 to -78 of the SAA2 promoter or only the individual binding sites), preferably in multimeric form.

In a preferred embodiment, a socalled positive feedback loop is achieved (Nettelbeck et al., Gene Therapy 5 (1998) 1656-1664). For this purpose, a gene is inserted downstream of the SAA2 promoter and of the therapeutically active gene, e.g., by means of coupling via an IRES sequence, which gene encodes a fusion protein consisting of a strong transcription activator, such as, e.g., HSV VP16, and a DNA binding protein, such as, e.g., LexA. A plurality of LexA binding sites to which the fusion protein can bind, thus activating the downstream promoter, are inserted upstream of the SAA2 promoter. After specific activation of the cytokine-inducible promoter, the therapeutic gene, and also the activating fusion gene, are expressed, and then the cytokine-inducible promoter is yet more strongly activated and the therapeutic gene and the activating fusion gene are yet more highly expressed, and so on.

A gene therapy agent according to the invention is understood to mean a pharmaceutical composition which contains one or more expression vectors according to the invention as essential components, in an amount needed by the tumor patient to ensure an effective treatment. Such a composition preferably contains at least one vector together with a non-viral delivery system, as an adenoviral vector or as a retroviral vector. In such cases, the delivery system or the viral vector per se or the expressed CD40/CD40L gene will cause a local or systemic inflammatory response in the tumor and/or in the tissue surrounding the tumor. In such cases, cytokine mobilization caused by the administration of the delivery/targeting vehicle would lead to the promoter-driven production of the therapeutic agent at up to at least a hundred times that which can be achieved using promoters which are not cytokine-inducible.

Gene therapy of somatic cells can be accomplished by using, e.g., retroviral vectors, other viral vectors or by non-viral gene transfer (cf. Friedman, T., Science 244 (1989) 1275; Morgan 1993, RAC Data Management Report, June 1993).

Vector systems suitable for gene therapy are, for instance, retroviruses (Mulligan, R.C., (1991) in Nobel Symposium 8: Etiology of human disease at the DNA level (Lindsten, J., and Pattersun, eds.) 143-189, Raven Press), adeno-associated virus (McLughlin, J. Virol. 92 (1988) 1963), Vaccinia virus (Moss et al., Ann. Rev. Immunol. 5 (1987) 305), bovine papilloma virus (Rasmussen et al., Methods Enzymol. 139 (1986) 642) or viruses from the group of the Herpes viruses, such as Epstein-Barr virus (Margolskee et al., Mol. Cell. Biol. 8 (1988) 2937) or Herpes simplex virus. However, adenoviruses are preferred.

There are also known non-viral delivery systems. For this, usually "nude" nucleic acid, preferably DNA, is used, or nucleic acid together with an auxiliary agent, such as, e.g., transfer reagents (liposomes, dendromers, polylysine transferrin conjugates (Felgner et al., Proc. Natl. Acad. Sci. USA 84 (1987) 7413).

It was found that it is possible according to the invention to create and activate tumor-specific T cells which are cytolytically active for a very long period of time. The combination of CD40 and CD154 expression, the combination of CD40 and/or CD154 expression with the expression of IL-2, IL-12 and/or Interferon-alpha or the combination of CD40 and/or CD154 expression with a therapeutically active amount of IL-2, IL-12 and/or Interferon-alpha polypeptides further lead to a synergistic enhancement of the level and duration of the activation phase of said tumor-specific T cells.

The pharmaceutical compositions may be administered parenterally, using, for example, injectable solutions, preferably for intratumoral injection into head and neck cancer (a squamous cell carcinoma). For the preparation of such injectable solutions, the vectors according to the invention are admixed with pharmaceutical inert, inorganic or organic excipients. Such excipients are, for example, water, phosphate buffered saline. pharmaceutically acceptable buffers (like phosphate, lactate, Tris), alcohols, polyols, glycerol, preferably having a neutral pH value (pH 6-8). The pharmaceutical compositions may also contain preserving agents, solubilizing agents, stabilizing agents, wetting agents, salts for the variation of osmotic pressure, buffers or antioxidants. They may also contain other therapeutically valuable agents.

Suitable preservatives for use in such preparations include benzalkonium chloride, benzethonium chloride, chlorobutanol, thimerosal, and the like. Suitable buffers include boric acid, sodium and potassium bicarbonate, sodium and potassium borates, sodium and potassium carbonate, sodium acetate, sodium biphosphate and the like, in amounts sufficient to maintain the pH between about pH 6 and pH 8, preferably between about pH 7 and pH 7.5. Suitable tonicity agents are dextran 40, dextran 70, dextrose, glycerine, potassium chloride, propylene glycol, sodium chloride, and the like. Suitable antioxidant and stabilizers include sodium bisulfite, sodium metabisulfite, sodium thiosulfate, thiourea and the like. Suitable wetting and clarifying agents include polysorbate 80, polysorbate 20, poloxamer 282, and tyloxapol. Suitable viscosity increasing agents include dextran 40, gelatin, glycerin, hydroxyethyl cellulose, hydroymethylpropyl cellulose, lanolin, methylcellulose, petrolatum, polyethylene glycol, polyvinyl alcohol, polyvinyl polyvinylpyrrolidone, carboxymethyl cellulose and the like.

The interaction between CD40 on antigen-presenting cells (APC) and its ligand CD154 on T cells exerts a prominent role in the upregulation of APC functions. These include the activation of B7 surface expression and IL-12 synthesis, two proteins which cooperate in the induction of an effective anti-tumor response. It was found that tumor cells from basal and squamous cell carcinoma exhibit a down-regulation or a dramatic loss of CD40 which may account for a tumor escape mechanism, by which activated T cells expressing CD154 are not able to kill the tumor cells any longer. The potential of CD40 in inducing an anti-tumor response can be shown by a stable transfection of a non-immunogenic CD40⁻ tumor cell (e.g., a squamous cell carcinoma cell line like SCCVII) with the murine CD40. Wild-type SCCVII, the neo control and CD40⁺ cells were indistinguishable in their proliferation rate and morphology in vitro. Syngeneic C3H/HeN mice injected s.c. with CD40⁺ SCCVII had a significant reduction in tumor growth compared to the CD40⁻ wild-type SCCVII or the neo control. In contrast, CD40-transfected cells injected into nude mice exerted the same tumor growth kinetic as the two controls indicating that immunocompetent T cells are necessary for anti-tumor activity. Histological analysis of tumor explants from CD40⁺ tumors revealed predominant infiltration of CD4⁺ T cells and expression of the CD25 T cell activation marker. These changes were not detectable in control group tumors. CD40 ligation induces apoptosis and inhibition of the proliferation rate in tumor cells transfected with CD40.

In accordance with the invention, the vectors according to the invention can be used in the control or treatment of tumor diseases, preferably in treatment of vascularized tumors. The dosage is a biologically effective amount of the vector and can vary within wide limits and is, of course, fitted to the individual requirements in each particular case. The preferable dose for viral vectors is 10⁸-10¹¹ pFU/injection and 50-1000 µg DNA/injection for nonviral vectors. The preferable volume per injection is between 1 and 10 ml.

The patient is preferably treated in such a way that 1-2 injections per week are administered directly into the tumor over a period of 3-10 weeks. After that period, the extent of change of the tumor is examined and, if necessary, as well as if possible, the tumor is removed. A therapy regimen of this kind is of particular importance with respect to improving the results of therapy, because after post-operative treatment, a sufficient number of tumor cells will still be present which are capable of inducing a cytotoxic immune response after transfection with the vectors according to the invention. This results in the formation of cytotoxic T cells which are capable of destroying metastasizing cells and cells of minimal residual disease.

It is further preferred to administer the individual injections into a plurality of sites in the tumor and/or in the vicinity of the tumor, which allows the pharmaceutical agent according to the invention to reach not only the actual tumor cells but also other cells of the tumor tissue, for example fibroblasts, macrophages, T cells or dendritic cells.

It is further preferred that prior to, during or directly after administration of the pharmaceutical agent according to the invention, the inflammatory reaction on the tumor should be intensified locally. This can be accomplished by means of, for example, local thermal treatment (microwaves), pressure, or by injection of the pharmaceutical agent into a plurality of sites in and in the vicinity of the tumor.

The vectors according to the invention may be injected as formulations with transfer reagents directly into tumors, post-operatively into tumor caves, or systemically.

In a preferred embodiment of the invention, the vector-containing pharmaceutical agent according to the invention (preferably containing no expression vector for IL-2, IL-12 and/or Interferon-alpha) is administered as an adjuvant and in combination with a polypeptide having the activity of IL-2, IL-12 and/or Interferon-alpha. In this case, the vector-containing agent is administered as described above, whereas the polypeptide is preferably administered systemically and before and/or after the injection of the vector-containing agent. In this connection, it is preferred to administer the polypeptide before and after the said injection.

Table 3 shows the preferred administration scheme for the polypeptides.

**Table 3**

| **Polypeptide** | **Injections per day** | **Amount per injection** | **Amount per day** |
|---|---|---|---|
| IL-2 | 1-2 | 0.6-2.5 10⁶ units | 0.6-5x10⁶ units |
| IL-12 | 1-2 | 1-2x10⁴ units | 1-4x10⁴ units |
| Interferon-alpha | 1-2 | 0.5-1x10⁶ units | 0.5-2x10⁶ units |

According to the invention, it is important to use endotoxin-free DNA and to avoid any inflammatory reactions of the tissue. This would induce preinflammatoy cytokine expression which on the other hand would lead to a stimulation of the inducible promoter in other tissues but squamous cell carcinomas. Such endotoxin-free DNA can be produced according to WO 97/29113.

For this treatment long lasting and high expression of the immune stimulatory gene is favourable as well as tumor cell specificity which would reduce side effects due to draining of the plasmids from the injected tumors into neighbouring tissues or other organs like liver or lung. Additionally tumor specific treatment could result in systemic injection of the tumor-targeted immune stimulatory plasmid-formulation. Tumor specificity can be achieved by either targeting of the transfection reagents to tumors or tumor-specific gene expression or combinations of both. Targeting of the transfection reagents is not yet feasible with sufficient specificity and efficiency but tumor specific promoters are available.

According to the description, this invention will have great impact on the treatment of tumors, metastases and minimal residual disease by vectors which code for immune stimulatory genes. The use of the promoters according to the invention allows high-level, long-lasting, potentially inducible/repressable and tumor specific expression. Therefore in cancer treatment regimens using vector DNA injection it could optimize the results due to longer lasting and higher amounts of the immune stimulatory proteins in the tumor, the possibility of systemic treatment which would reach not only the primary tumors but also metastases, and a wider therapeutic window (higher doses and multiple dosing) due to reduced toxic side effects.

The following examples, references and figures are provided to aid the understanding of the present invention, the true scope of which is set forth in the appended claims. It is understood that modifications can be made in the procedures set forth without departing from the spirit of the invention.

### Description of the Figures

- **Figure 1**: Transfection experiments of the plasmid pSAA2-CAT with the SAA2-promoter driving the expression of the Cat-gene and as control the plasmid pCMV-CAT with the CMV immediate early promoter.
- **Figure 2**: Experiments differing from those shown in Figure 1 by the reduction of the incubation time after the induction and by the use of only 2 cytokines (IL-1β and IL-6) for induction.
- **Figure 3**: Determination of the induction of the SAA2-promoter only by IL-1β.
- **Figure 4**: FACS analysis of SCC wild-type, SCC neo transfectant and two CD40 transfected SCC cells. Both CD40 transfected clones show significant expression of CD40.
- **Figure 5**: In vivo growth kinetics of SCC CD40, SCC neo transfectant clone and SCC wt (wild-type) cells. Syngenic C3H/HeN mice (n = 10) were inoculated with 5 x 10⁵ cells s.c. and tumor growth was monitored 3 times a week using calliper. Tumor volume was calculated using ellipsoid formula 4/3 x π/8 x (I x w x d). SCC CD40 transfectant shows a significant reduction of growth compared to SCC neo control and SCC wild-type cells (p < 0.01). Error bars represents SD (standard deviation).
- **Figure 6**: In vivo growth kinetics of SCC CD40, SCC neo transfectant clone and SCC wt in Balb/c nude mice. 5 x 10⁵ cells were inoculated s.c. in athymic Balb/c nu/nu mice (n = 10) and tumor volume was measured as described in Fig. 5. Error bars represents SD.

### Materials and Methods

### Animals:

Female syngeneic C3H/HeN (H-2K^{k}) mice were purchased from Charles River, Germany. The mice were between 8-12 weeks old. Female Balb/c nu/nu mice (20 weeks old) were obtained from Bomholtgard Breeding, Denmark.

### Cell lines:

SCCVII/SF cells were cultured in DMEM high glutamax® medium (GIBCO Life Technologies) supplemented with 10% heat-inactivated FCS (GIBCO Life Technologies) penicillin and streptomycin (Roche Diagnostics GmbH, Germany).

### DNA constructs and transfection:

The full length cDNA for murine CD40 was cloned into the pcDNA3 (Invitrogen) mammalian expression vector. Transfection of SCCVII cells was carried out using calcium phosphate (Invitrogen) or DOTAP (Roche Diagnostics GmbH, Germany). Resistant clones were selected with neomycin (G 418, Roche Diagnostics GmbH, Germany) for 14 days at a concentration of 500 mg/l. Clones were analyzed for CD40 expression by FACScan® (Becton Dickinson, Germany) and sorted for high expression using a FACSorter® (Becton Dickinson, Germany). For neo mock control, SCCVII/SF cells were transfected with the pcDNA3 expression vector alone.

### Flow cytometry and antibodies:

Cells were labeled by direct immunofluorescence using phycoerythrin (PE) conjugated monoclonal antibodies (mAbs) against MHC class I H-2K^{k} (clone 36-7-5, Pharmingen) and CD40 (clone 3/23, Pharmingen). PE conjugated isotype control (Pharmingen) was used for determination of unspecific binding. Anti-MHC class II I-A^{k} and I-E^{k} (Pharmingen) mAb, fluorescein isothiocyanate (FITC) conjugated, were applied together with an FITC isotype control. Anti-Fas and anti-FasLigand (Dianova) directed mAb were PE direct conjugated. Antibody staining was carried out for 30 min. at 4°C followed by washing and analyzing using a FAScan® (Becton Dickinson, Germany).

### In vivo experiments:

Tumor cells were inoculated in a concentration of 5 x 10⁵/200 µl subcutaneously (s.c.) in the right hind flank of syngenic or athymic animals. Tumor growth was quantitated five days post-inoculation three times a week using a calipper. Tumor volumes were calculated by the approximately ellipsoid formula: 4/3 x π / 8 x (length x width x depth).

### Example 1

### Construction of expression vectors

a) pSAA2-Cat was constructed by insertion of the SAA2-promoter (1218 bp sequence; Uhlar et al., J. Immunol. Methods 203 (1997) 123-130) as a BamHI / NsiI fragment into pCMV-H12-Bgh-Cat, from which the human CMV-promoter was deleted by PstI / BamHI restriction.
b) pCMV-H12-Bgh-Cat construction: The β-galactosidase-gene from pCMV-β (CLONTECH) was removed by partial digestion with MluI/NotI and isolation of a 3,7 kb vector fragment; the Bgh polyadenylation signal was taken from pCDNA3 (Invitrogen); the SV40-poly-A site was also removed from pCMV-β; the hybrid intron H12 was taken from pAD-β (CLONTECH); and the Cat gene was taken from pSV2-Cat. All these fragment were put together to give: human CMV-promoter/enhancer- H12 intron - Cat gene - Bgh-poly-A; the vector backbone is from pUC-vectors with the pUC-origin of replication and an Ampicillin resistance gene.
c) pSAA2-CD40L was constructed by removing of the Cat gene in pSAA2-Cat by NotI-digestion, and cloning of the mCD40-L cDNA which was amplified by PCR using primers with NotI-overhangs and restriction with NotI.
d) pSAA2-CD40 was constructed by removing of the Cat gene in pSAA2-Cat by NotI-digestion, and cloning of the mCD40 cDNA which was amplified by PCR using primers with NotI-overhangs and restriction with NotI.
e) pSAA2-IL-2 was constructed by removing of the Cat gene in pSAA2-Cat by NotI-digestion, and cloning of the IL-2 cDNA which was amplified by PCR using primers with NotI-overhangs and restriction with NotI.
f) pSAA2-IL12 was constructed by removing of the Cat gene in pSAA2-Cat by NotI-digestion. The 2 subunit-genes p35 and p40 of the IL-12 were separately amplified by PCR. Their transcription was coupled by the use of an IRES sequence from EMCV (derived from pIRESlneo; Clontech). P35 was amplified by a 5'-primer with a NotI-site in the overhang and a 3'-primer with a SalI-site in the overhang. EMCV IRES was amplified by a 5'-primer with a SalI-site in the overhang and a 3'-primer with a XhoI-site in the overhang. P40 was amplified by a 5'-primer with a XhoI-site in the overhang and a 3'-primer with a NotI-site as well as a XhoI-site in the overhang. The amplified fragments were then successively cloned into pBluescript (Stratagene) which was cut by NotI and XhoI. The final plasmid pBluescript-p35-IRES-p40 was then cut with NotI and the fragment containing p35 and p40 transcriptionally coupled by EMCV IRES was cloned into pSAA2-Cat instead of the Cat-gene.
g) AdV-SAA2-CD40L:

Adenovirus vectors are constructed by cloning the genes to be expressed into an Adenovirus transfer vector which contains the expression cassette and regions homologous to the Adenovirus genome. The AdV transfer vector and the AdV genome are linearised and cotransfected into 293A cells. There they recombine to form AdV vectors which can be packed into viral particles but cannot replicate due to deletions in their E1 and E3 regions. This deletions result in the cloning capacity of 7 kb. The AdV vectors can only replicate and therefore be produced in 293A cells which are stable transfected by sheared AdV DNA and therefore provide the E1 and E3 gene products in trans. The Adenovirus vector AdV-SAA2-CD40L was constructed by insertion of the whole expression cassette from pSAA2-CD40L into the Adenovirus transfer vector pQBI-AdCMV5 (QUANTUM Biotechnologies Inc.) from which the CMV5 promoter / enhancer and the globin-poly-A site have been removed. Due to the lack of suitable restriction sites the expresssion cassette from pSAA2-CD40L was amplified by PCR and also the pQBI-AdCMV5 vector without the CMV5 promoter/enhancer and the globin polyA was amplified by PCR. The 2 amplified DNA fragments were restricted with Pmel (the recognition sites were provided by the overhangs of each PCR-primer) and ligated by T4-DNA ligase. The Adenovirus transfer vector AdV-SAA2-CD40L was linearised by Clal digestion at the linearization site and cotransfected with linear QBI-viral DNA into QBI-293A cells according to manufacturer's protocol (QUANTUM Biotechnologies Inc.). In the 293A the transfer vector and the cotransfected adenoviral DNA recombine in their overlapping regions to form the Adenovirus vector AdV-SAA2-CD40L. AdV-SAA2-CD40L has deletions in the E1 and E3 region of human Adenovirus type 5; the SAA2-CD40L expression cassette is cloned instead of the deleted El region. Due to the E1 deletion the Adenovirus vector is not able to replicate alone. Only in 293A cells which contain the sheared genomic DNA from human Adenovirus type 5, the E1A and E1B genes which are essential for virus replication are provided in trans and therefore the virus could be produced in that cell line. The virus vector was purified via several cycles of plaque purification and isolated in large scale from the infected 293A cells by methods described in the QUANTUM Adeno-Quest Kit protocol or reviewed by Graham et al., Mol. Biotechnol. 3 (1995) 207 - 220.
h) The adenovirus vector AdV-SAA2-CD40 was constructed analogous AdV-SAA2-CD40L by using the expression cassette of pSAA2-CD40 instead of pSAA2-CD40L.
i) The adenovirus vector AdV-SAA2-IL-2 was constructed analogous AdV-SAA2-CD40L by using the expression cassette of pSAA2-Il-2 instead of pSAA2-CD40L.
k) The adenovirus vector AdV-SAA2-IL-12 was constructed analogous AdV-SAA2-CD40L by using the expression cassette of pSAA2-IL-12 instead of pSAA2-CD40L.

### Example 2

### Induction of SAA2-promoter by cytokines

The inducibility of the human SAA2-promoter by the proinflammatory cytokines IL-6, IL-1β, and TNF-α could be reproduced in the HepG2 cell line as well as in the SCC-7 cell line.

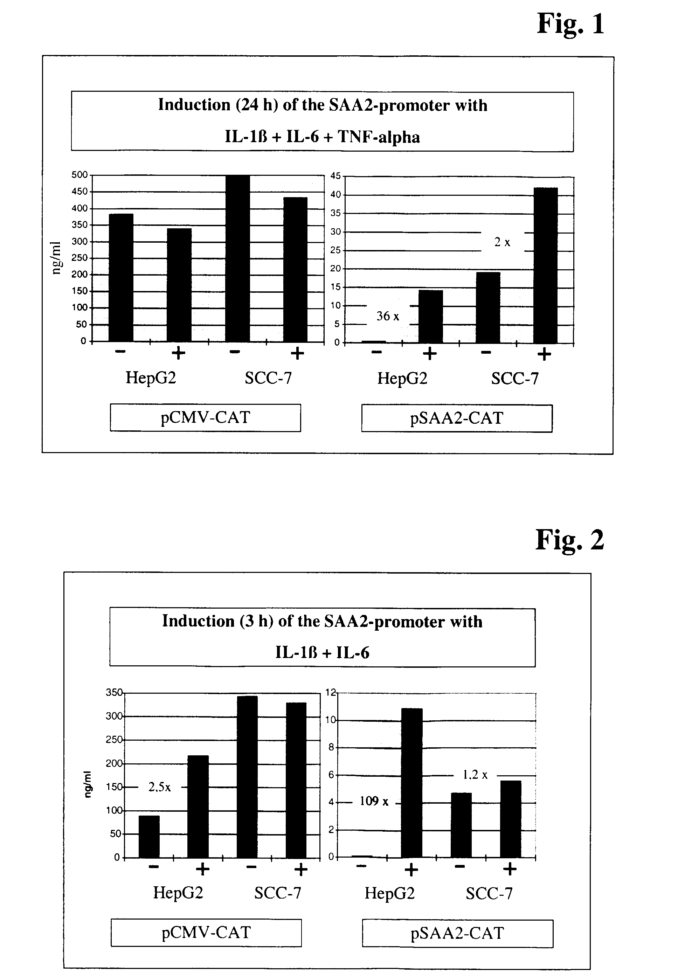

Figure 1 shows transfection experiments of the plasmid pSAA2-CAT with the SAA2-promoter driving the expression of the Cat-gene and as control the plasmid pCMV-CAT with the CMV immediate early promoter. Confluent HepG2 or SCC-7 cells in 6-well plates were transfected by 2 µg plasmid and incubated for 24 h. After that time they were stimulated (+) by the addition of 10 ng /ml cytokine (Il-1β, IL-6, TNF-alpna) or not stimulated as control (-). Experiments were done in duplicates. 24h later the Cat-gene expression was determined by Cat-ELISA on several dilutions (1:1, 1:10, 1:100) and the amount of Cat was calculated in [ng/ml].

Figure 1 shows that the CMV-promoter is uninducible by IL-1β, IL-6, TNF-alpha in both cell lines, however its expression rate is much stronger (30x in HepG2; lOx in SCC-7) than the fully stimulated SAA2 promoter. There is nearly no expression of the uninduced SAA2-promoter in HepG2-cells. Uninduced SCC-7 cells show a much higher (50x) basal expression of the Cat-gene than uninduced HepG2 cells. The expression of the the SAA2-promoter is inducible by the proinflammatory cytokines used, however the inducibilty is much higher (36x versus 2x) in HepG2 than in SCC-7 cells.

Consequently, the squamous carcinoma cell line SCC-7 secrets own proinflammatory cytokines leading to an autostimulation of the SAA2-promoter. Therefore the addition of the inducing cytokines lead to a much stonger induction of the SAA2-promoter in HepG2 than in SCC-7 cells.

Figure 2 shows a 2,5-fold induction of the CMV-promoter in HepG2 cells by the combination of IL-1β + IL-6 versus no induction in HepG2 cells by IL-1β + IL-6 + TNF-alpha (this effect was verified in 3 separate experiments but there is no explanation available why such induction could be seen here). The induction rate of the SAA2-promoter in HepG2 with IL-1β + IL-6 is very strong (109x) leading to comparable expression levels of cat induced with IL-β + IL-6 + TNF-alpha (Figure 1). According to Figure 1 the expression level of the uninduced SAA2-promoter in HepG2 cells is nearly zero. The basal level of the uninduced SAA2-promoter is at the same amount (50-fold) higher in SCC-7 cells than in HepG2 cells. Only the induction rate of the SAA2-promoter in SCC-7 cells is lower (only 1,2x versus 2x). This can be due to the additional stimulatory activity of TNF-alpha in the experiment described in Figure 1 or due to kinetics of the induction (the ELISA in Figure 1 was performed 24 h after induction, the ELISA in Figure 2 only 3 h after induction). The result was meassured 24 h after induction by Cat-ELISA.

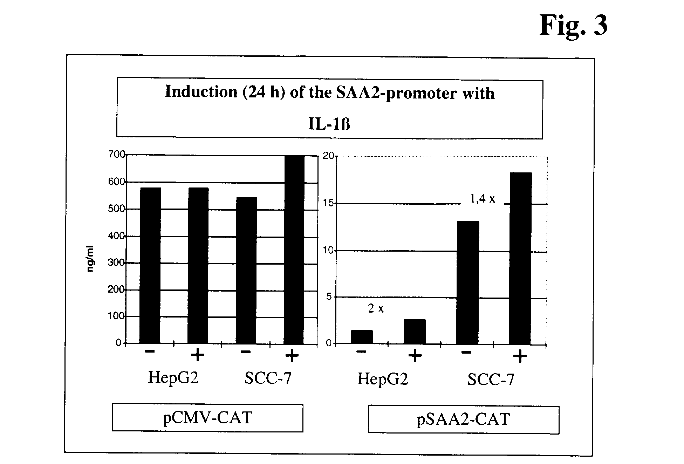

Figure 3 shows no induction of the CMV-promoter by IL-1β as already seen in Figure 1 with the cytokines IL-1β + IL-6 + TNF-alpha. The differences in basal expression levels are the same as in Figures 1 and 2 (nearly zero in HepG2, 10-fold higher in SCC-7). However there is only a very weak induction of the SAA2-promoter by IL-β detectible in both cell lines after 24h (1,4x for SCC-7 and 2x for HepG2). This result stands against data from Uhlar et al., J. Immunol. Methods 203 (1997) 123-130, who described a very strong induction of the SAA2-promoter by the action of the cytokine IL-1β.

### Conclusion

The experiments described above show that the CMV-promoter is 1-2 logs stronger than the induced SAA2 promoter. The SAA2 promoter is silent in the absence of inducing proinflammatory cytokines. Proinflammatory cytokines like Il-1β, IL-6 or TNF-alpha can induce the SAA2 promoter. The squamous carcinoma cell line SCC-7 seems to express one or more of these cytokines in vitro leading to an induction of the SAA2 promoter, giving a higher basal expression level of the SAA2 promoter than the other cell line HepG2.

### Example 3

### In vitro growth and surface expression of MHCI and CD40

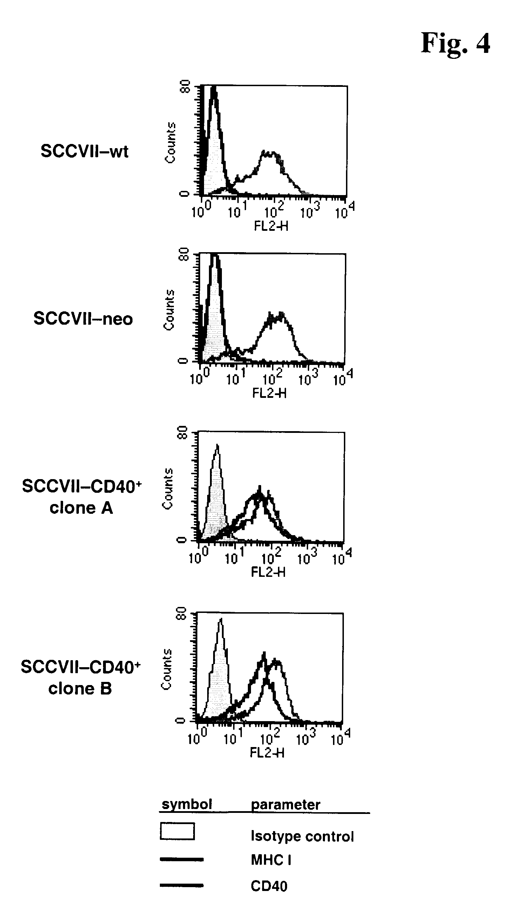

Two transfectants expressing CD40 were chosen for further investigation. Both of them show similar expression levels of CD40. No expression of the co-stimulatory molecule B7.1, MHCII and FasLigand could be found. The two clones show similar morphology and in vitro growth kinetic to those of SCC wild-type (wt) cell line and SCC neo transfectant clones (Fig. 4).

### Example 4

### In vivo tumor growth in syngenic mice

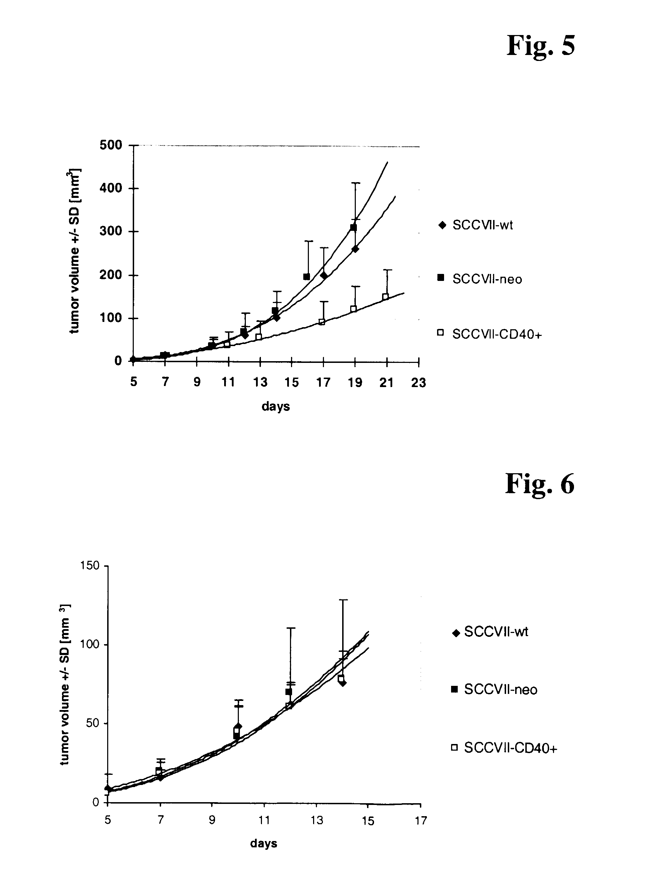

To examine the tumor growth kinetic of the SCC CD40 transfectant, the SCC neo control clone and the SCC wt cells, cells were inoculated at a concentration of 5 x 10⁵ cells per mouse s.c. into the right hind flank of syngenic C3H/HeN mice. The SCC CD40 clone grew in vivo with a significant (p < 0.001) growth reduction (Fig. 5). The neomycin control transfectant clone grew similar to the wild-type (wt) cell line. A further experiment including two individual SCC CD40 expressing clones showed a similar in vivo growth reduction with significant reduction for both clones (p < 0.01).

### Example 5

### In vivo tumor growth in athymic mice

In order to demonstrate whether T cells play a pivotal role for the in vivo tumor growth reduction, the Balb/c nu/nu athymic mice strain which lacks mature T cells was used. SCC wt cell line, SCC neo control and SCC CD40 expressing clones were inoculated at the same pattern as described for syngenic C3H/HeN mice. The growth kinetic was measured using calipper starting at day 5 for every second or third day. All inoculated clones showed a similar growth rate in the nude mice strain (Fig. 6).

### Example 6

### In vivo tumor growth in C3H/HeN mice bearing squamous cell carcinoma

Squamous cell carcinoma (SCC) cells (5x10 exp 6/0.2 ml) are injected s.c. into C3H/HeN mice. Ten to 15 days thereafter when tumors have grown to an appropriate size, AdCMV-mCD40L (5 x10⁸/50 µl) are injected intra-tumorally. Tumor volume is measured every third day for 21 days and compared to tumors of mice injected with the control AdCMV-LacZ. After termination of the experiment, histopathology is performed with explanted tumors and checked for activated T cells (CD4, CD25). In addition, dose response curves with different concentrations of viral vector (1x10⁷ to 1x10⁹) and comparison of single vs. multiple injections are performed.

From these studies, the optimal regimen can be established. In the case of tumor regression, treated mice are challenged with tumor cells i.p. into the contra-lateral side. Growth of secondary tumor is measured to monitor memory response. In a second tumor model, CT26 (colon) tumor cells are used to extend this approach to a broader basis.

Subsequent studies show the synergistic potential of AdCMV-CD40L in combination with AdCMV-IL12 in the preclinical tumor models.

### List of References

Beach, C.M., et al., Biochem. J. 282 (1992) 615

Birt, D.F., et al., J. Nutr. 129 (1999) 25 Suppl., 5715-5745

Conary et al., J. Clin. Invest. 93 (1994) 1834-1840

Dendorfer, U., Artif. Organs 20 (1996) 437-444

Dwulet, F.E., et al., Biochemistry 27 (1988) 1677

Felgner et al., Proc. Natl. Acad. Sci. USA 84 (1987) 7413

Fey, G.H., and Gauldie, J., In: H. Popper and F. Schaffner (Eds.), Progress in Liver Diseases. Vol.9. (1989) WB Saunders, Philadelphia, p.89

Friedman, T., Science 244 (1989) 1275

Graham et al., Mol. Biotechnol. 3 (1995) 207 - 220

Grimaldi et al., The J. of Immunol. 149 (1992) 3921-3926

Hoffman, J.S., and Benditt, E.P., J. Biol. Chem. 257 (1982) 10510

Kang, D.C., et al., Int. J. Oncol. 13(1998) 1117-1126

Knerer et al., Acta Oto-Laryngologica 116 (1996) 132-136

Kushner, I., and Mackiewicz, A., Disease Markers 5 (1987) 1

Kushner, I., Ann. NY Acad. Sci. USA 389 (1982) 39

Laman et al., Critical Reviews in Immunology 16 (1996) 59-108

Levy et al., Neurosurgery 39 (1996) 823-823

Luckow, B., et al., Nucleic Acids Res. 10 (1987) 5490

Margolskee et al., Mol. Cell. Biol. 8 (1988) 2937

Marhaug, G., Scand. J. Immunol. 18 (1983) 329

McLughlin, J. Virol. 92 (1988) 1963

Morgan 1993, RAC Data Management Report, June 1993

Moss et al., Ann. Rev. Immunol. 5 (1987) 305

Mulligan, R.C., (1991) in Nobel Symposium 8: Etiology of human disease at the DNA level (Lindsten, J., and Pattersun, eds.) 143-189, Raven Press

Needleman and Wunsch, J. Mol. Biol. 48 (1970) 443-453

Nettelbeck et al., Gene Therapy 5 (1998) 1656-1664

Rasmussen et al., Methods Enzymol. 139 (1986) 642

Sambrook et al., "Expression of cloned genes in E.coli" in Molecular cloning: a laboratory manual (1989), Cold Spring Harbor Laboratory Press, New York, USA, 9.47-9.63 and 11.45-11.61

Sen, C.K., FASEB J. 10 (1996) 709-720

Sipe, J.D., et al., Biochemistry 24 (1985) 2931

Smith and Waterman, Adv. Appl. Math. 2 (1981) 482-489

Spear, B.T., et al., DNA Cell Biol. 14 (1995) 635-642

Stamenkovic et al., The EMBO J. 8 (1989) 1403-1410

Steel et al., Biochem. Journal 291 (1993) 701-707

Steel, D.M., and Whitehead, A.S., Immunol. Today 15 (1994) 81

U.S. Patent No. 5,744,304

U.S. Patent No. 5,851,822

Uhlar et al., J. Immunol. Methods 203 (1997) 123-130

van Kooten et al., Int. Arch. Allergy Immunol. 113 (1997) 393-399

Varley, A.W., Proc. Natl. Acad. Sci. USA 92 (1995) 5346-5356

Weber-Nordt, R.M., et al., Leuk. Lymphoma 28 (1998) 459-467

WO 93/08207

WO 93/08207

WO 97/29113

WO 98/40506

Woo, P., et al., J. Biol. Chem. 262 (1987) 15790-15795

## Claims

1. A vector for the expression of a nucleic acid encoding at least one polypeptide with CD40 and/or CD154 activity, wherein the expression of said nucleic acid is under the control of a cytokine-inducible promoter.

2. The vector according to claim 1, said vector containing in addition a nucleic acid encoding IL-2, IL-12 and/or Interferon-alpha.

3. The vector according to claim 1 or 2, wherein the promoter is a human acute phase amyloid A gene promoter.

4. The vector according to claims 1 to 3, wherein said vector is an adenoviral vector.

5. A pharmaceutical composition containing an expression vector according to claims 1 to 4 as an essential component.

6. The pharmaceutical composition according to claim 5, wherein the said composition contains the vector in an amount of 10⁸-10¹¹ pFU.

7. A method for the production of a pharmaceutical composition for the treatment of tumors, wherein a vector according to claims 1 to 4 is used as an essential component of the composition.

8. A method for the treatment of a patient for tumor therapy, wherein the patient is treated by the use of the pharmaceutical composition according to claim 5 or 6.

9. A method for the treatment of a patient for tumor therapy, wherein the patient is treated by the use of the pharmaceutical composition containing an expression vector according to claim 1 and with Interleukin-2, Interleukin-12, Interferon-alpha and/or 5-fluorouracil.

10. A substance for use in a method of treatment for tumor therapy, said substance comprising a vector according to claims 1 to 4 and said method comprising administering said vector to the patient in need of such treatment.
